(19) Europäisches Patentamt

European Patent Office

Office européen des brevets

(11) **EP 1 004 872 A1**

(12) **EUROPEAN PATENT APPLICATION**

(43) Date of publication:
**31.05.2000 Bulletin 2000/22**

(51) Int. Cl.⁷: **G01N 21/55**, G01N 21/43,
G01N 33/03

(21) Application number: **99203913.1**

(22) Date of filing: **23.11.1999**

(84) Designated Contracting States:
**AT BE CH CY DE DK ES FI FR GB GR IE IT LI LU
MC NL PT SE**
Designated Extension States:
**AL LT LV MK RO SI**

(30) Priority: **23.11.1998 US 109558 P**

(71) Applicant:
**Texas Instruments Incorporated
Dallas, Texas 75251 (US)**

(72) Inventors:
• **Carr, Richard A.
Rowlett, Texas 75088 (US)**
• **Melendez, Jose L.
Plano, Texas 75025 (US)**
• **Elkind, Jerome L.
Richardson, Texas 75081 (US)**

(74) Representative:
**Holt, Michael et al
Texas Instruments Limited,
European Patents Department (MS 13),
PO Box 5069
Northampton NN4 7ZE (GB)**

(54) **System and method for sensing the quality of a medium**

(57)     The present invention comprises a system for and method automatically monitoring and improving oil quality, comprising a container of oil (10), such as a vat of frying oil, an inlet (20) for drawing a sample of the oil from the container of oil, a temperature control system (24), a fixed optic sensor (34), and a digital signal processing unit (14). The system of the present invention may optionally comprise a heating device for adjusting the temperature of the fixed optic sensor (34) to a temperature substantially equivalent to the temperature of the sample, a container of at least one reagent (26) for reacting with the sample, a container of at least one cleaning agent (95) for cleaning the surface of the fixed optic sensor (34), an audio/visual alarm (56) for indicating that the quality of the oil has degraded to an unacceptable level, at least one disposal unit (66, 90) for collecting the sub-par quality oil and the sample after analysis, a container of fresh oil (76) for adding fresh oil to the container of oil, a variable control input (50) for adjusting the level at which the quality of the oil is deemed unacceptable, a local data storage medium (99) for recording data, and a remote data storage medium for receiving data elements transmitted via a network.

FIGURE 1

EP 1 004 872 A1

## Description

FIELD OF THE INVENTION

The present invention relates in general to sensor systems in the fields of chemical, biochemical, biological and biomedical analysis, and more particularly, to a system, method and apparatus for sensing the quality of a medium such as cooking oil.

BACKGROUND OF THE INVENTION

Without limiting the scope of the present invention, its background is described in connection with monitoring and improving the quality of fluids such as oil used in frying or cooking foods. It should be appreciated by one skilled in the art that the term cooking oil or oil refers to a variety of oils and other media, and that the principles of the present teachings are applicable to a variety of media.

In conventional deep frying, foods are immersed in a frying medium heated to temperatures typically in the range of 160 to 180 degrees Celcius. Frying is a dehydration process in which the hot frying medium, e.g., cooking oil, transfers heat to the food product by heating water within the product and driving the water out into the oil as vapor. As the frying process begins, materials such as water, oxygen, heat, salts, metals, food colorants, and breaded materials start a chain reaction of degradation, converting the oil into a mixture of frying components and contaminants.

Such degradation of the cooking oil due primarily to oxidation and hydrolysis produces greater levels of monoglycerides, diglycerides, free fatty acids, ketones, glycerine, and other harmful compounds. These compounds are undesirable in view of the health and safety risks with which they are associated. In addition, foods cooked in poor quality oil have an unpleasant discoloration, taste and odor. Continued use of the same supply of cooking oil therefore significantly reduces the quality of the cooking oil. Thus, used cooking oil should be discarded when continued use renders the quality of the oil unacceptable.

SUMMARY OF THE INVENTION

In the fast-food restaurant industry, the discarding and replenishing of cooking oil are typically performed manually after a visual inspection of the used oil. The decision to discard and replace the oil is a highly subjective one, and is typically based on an individual's perception of the color, odor, or taste of the food cooked in the oil, or of the oil's color or amount of particulate matter. Such a subjective method is highly dependent on individual preferences and is susceptible to human error, and is therefore inherently unreliable and inaccurate. Additionally, such a method relies heavily on human involvement and requires training of personnel.

Restaurants in the fast-food industry typically do not provide substantial training to their employees in part due to the high turnover rate in the industry.

The prior art methods are also inefficient and costly, due to their time-consuming nature and their potential to make inefficient use of resources of oil by changing used oil on an unnecessarily frequent basis. In addition to increasing costs associated with personnel and oil supply, the generation of excess waste as a result of inefficient use of resources has an adverse impact on the environment.

Various assay kits and experimental tests have been made commercially available for measuring the quality of cooking oil. These experimental tests have typically utilized measurements of free fatty acid levels, total alkaline levels, polar compound levels, polymer levels, color readings, resistivity, changes in dielectric constant, and smoke point as indicators of the quality of the oil. These prior methods typically require a significant level of human involvement and skill, and are therefore impractical in the typical fast-food restaurant setting. Moreover, many of these assay kits require manual calibration. Furthermore, many of the devices in the prior art are incompatible with previously manufactured fryers.

A method and apparatus for measuring quality of cooking oil by monitoring changes in the capacitance and optical transmission of the oil is described in U.S. Patent No. 5,818,731, issued to Mittal et al. A disadvantage to this method is that it requires immersion of the sensor in the oil in the frying temperature range. In addition, this method lacks accuracy since its measurements of optical transmission are susceptible to error due to color changes in the oil as well as bubbles and particulate matter that interfere with transmission. Furthermore, the multi-plated parallel capacitor of the Mittal apparatus is very bulky and difficult to keep clean to ensure accurate measurements.

A need has therefore arisen for a system and method for sensing the quality of oil that overcomes the limitations in the prior art. A system and method that provide for accurate measurements of oil quality would have great advantages over the prior art. A system that may be retrofitted to existing fryers and that can interface with existing fryer control systems would be highly desirable as well.

The present application discloses a method of automatically monitoring and improving oil quality, comprising the steps of drawing a sample of oil, modifying the temperature of the sample of oil, passing the sample of oil onto a sensing surface of a fixed optic sensor, and optically sensing one or more properties of the sample of oil. The present application further comprises a system for automatically monitoring and improving oil quality, comprising a container of oil, such as a frying vat of oil, an inlet for drawing a sample of the oil from the container of oil, a temperature control system, a fixed optic sensor, and a digital signal processing unit. The disclosed system of the present application may optionally comprise a heating device, a container of one or more reagents, a container of one or more cleaning agents, an audio/visual alarm, one or more disposal units, a container of fresh oil, a variable control input, a local data storage medium for recording data, and a remote data storage medium for receiving one or more data elements transmitted via a network.

BRIEF DESCRIPTION OF THE DRAWINGS

For a more complete understanding of the embodiments disclosed by present application, including features and advantages, reference is now made to the following detailed description, taken in conjunction with the accompanying drawings in which:

> Figure 1 depicts a block diagram of a system for sensing the quality of cooking oil;
> Figure 2 depicts a schematic diagram of an exemplary fixed optic surface plasmon resonance sensor;
> Figure 3 depicts a schematic diagram of an exemplary fixed optic critical angle sensor; and
> Figure 4 depicts a flow diagram for a method of sensing and improving the quality of cooking oil.

Corresponding numerals and symbols in the different figures refer to corresponding parts unless otherwise indicated.

DETAILED DESCRIPTION OF THE PREFERRED EMBODIMENT

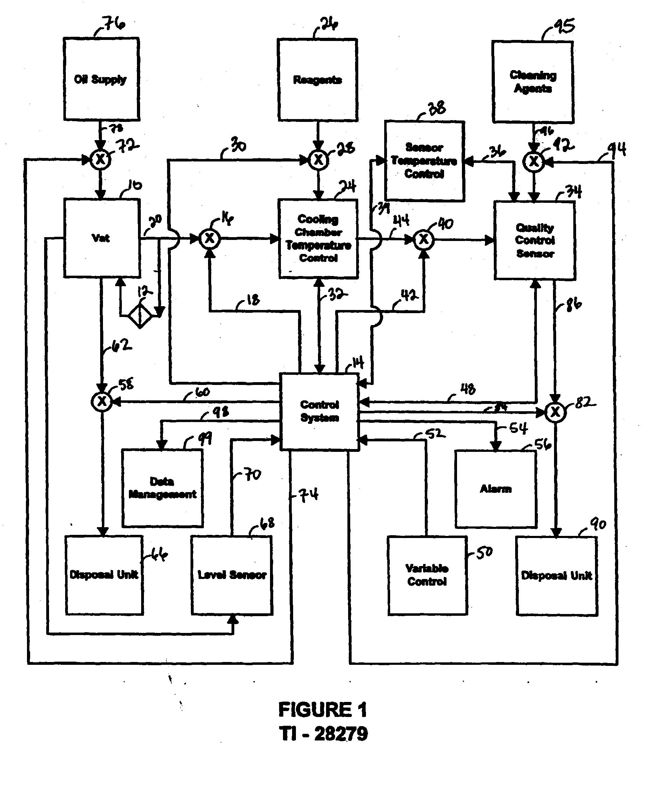

Reference is now made to Figure 1 in which a block diagram of a system for sensing the quality of a medium such as cooking oil in accordance with one embodiment of the present application is depicted.

A vat 10 contains cooking oil heated to a temperature suitable for cooking or frying foods. Vat 10 may comprise a conventional gas or electric deep fryer of a type commonly used in the restaurant industry. Oil may optionally pass through a filter 12 for removing particulate matter from the oil in vat 10.

A control system 14 is coupled to an electrically operated valve 16 via an electrical interface 18. Control system 14 may comprise a microcontroller or digital signal processing unit, such as TMS320F206 or TMS320F243, manufactured by Texas Instruments. When control system 14 opens electrically operated valve 16, a sample of the cooking oil in vat 10 is drawn into an inlet or flow line 20. Valve 16 is open for a period of time such that a volume of the sample suitable for analytical measurements is released from vat 10.

The sample enters a cooling chamber 24 where its temperature is monitored by a thermocouple or other temperature measuring device. One or more solutions may have been optionally added to cooling chamber 24 by opening an electrically operated valve 28 via a signal from control system 14 transmitted via an electrical interface 30. Solution 26 may be a cleaning agent such as a solvent or detergent or a reference or standard such as fresh, unused oil. Alternatively, solution 26 may be added to the sample to make the sample more conducive to analysis. For example, solution 26 may lower the viscosity of the sample.

The sample is cooled to a temperature that is generally above room temperature and is suitable for testing while maintaining a suitable level of viscosity such that the sample does not tend to solidify. The temperature of the sample may be tailored according to the specifications of the user, the composition of the sample, and other factors. For a typical cooking oil heated in the range of approximately 180 to 190 degrees Celcius, the temperature of the sample may be lowered to a temperature in the range of approximately 40 to 70 degrees Celcius, for example.

Control system 14 reads data indicative of the temperature of the sample via an electrical interface 32. The temperature of a quality control sensor 34 is monitored via an interface 36 to a temperature control system 38. Temperature control system 38 may comprise a thermocouple or a thermistor coupled with a heater such as a heating coil or heat lamp. Temperature control system 38 communicates with control system 14 via an interface 39. Temperature control system 38 adjusts the temperature of quality control sensor 34 to be approximately equivalent to the temperature of the sample to prevent condensation, solidification or other thermodynamic reaction.

When the temperature of the sample has cooled to a desired predetermined level, and the temperature of quality control sensor 34 has been adjusted accordingly, control system 14 opens an electrically operated valve 40 via an electrical interface 42. The sample is then released from flow line 44 to a sensing surface of quality control sensor 34, for the measurement of properties of the sample, namely changes in the refractive index of the sample.

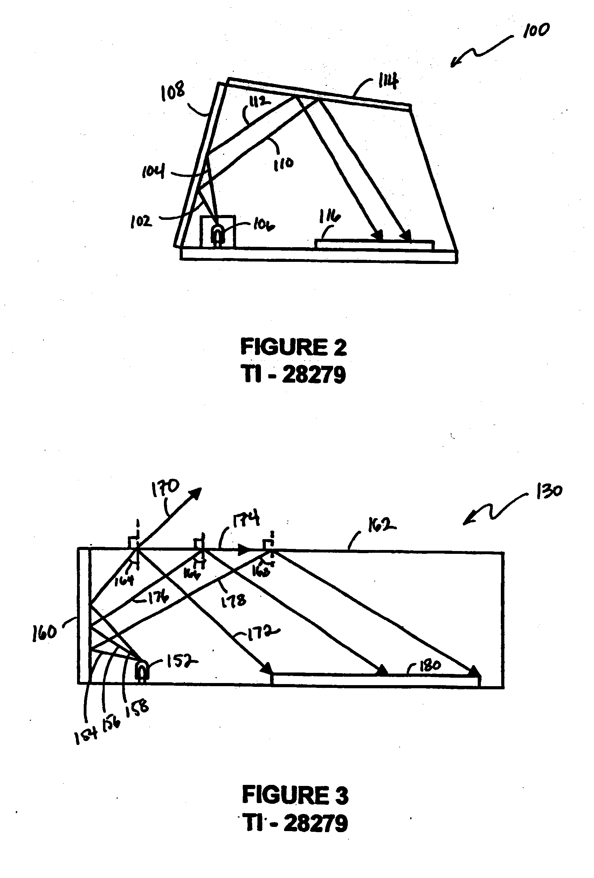

Quality control sensor 34 is preferably a surface plasmon resonance fixed optic sensor generally depicted as 100 in Figure 2. A surface plasmon, as known in the art, is a surface charge density wave at the surface of a dielectric interface having a thin conductive film formed thereon. The oscillation of free electrons at a conductor-dielectric boundary is affected by the refractive index of the material adjacent to the film. Using a polarized beam of monochromatic light, surface plasmon polaritons can be excited. Resonance occurs when the polarized light is totally internally reflected from the conductive film. The light internally reflected from the film has a minimum intensity at the resonance angle. By detecting the resonance angle, the refractive index of a material adjacent to the film may be determined, which is indicative of other properties of the material. A more detailed description of surface plasmon resonance may be found in the article "Surface Plasma Oscillations and Their Applications," Rather, H., Physics of Thin Films, 1977.

In accordance with fixed optic surface plasmon resonance sensor 100, polarized light rays 102 and 104 emanating from a monochromatic light source 106 strike a sensing surface 108. The precise composition of sensing surface 108 may be tailored according to the specifications of the user and the composition of the oil being tested. Components 110 and 112 of the light rays 102 and 104 may be transmitted from the sensing surface 108 onto a reflective surface 114, and are then reflected onto a photodetector 116. The reflective surface 114 may be a mirror that is flat, or may be concave or convex.

For measuring optical radiation, a suitable photodetector 116 has an array of discrete photosensing areas, or pixels. Light energy striking a pixel generates electron-hole pairs in the region under the pixel. The field generated by the bias on the pixel causes the electrons to collect in the element while the holes are swept into the substrate. Each sensing area in photodetector 116 thereby produces a signal on an output with a voltage that is proportional to the intensity of the light striking photodetector 116. This intensity and its corresponding voltage are at their maxima in the total internal reflection region. When fixed optic surface plasmon resonance sensor 100 serves as quality control sensor 34 of Figure 1, the output, representing bit level data from photodetector 116, is transmitted to control system 14.

Alternatively, quality control sensor 34 may be a refractometer such as a fixed optic critical angle sensor generally depicted as 130 in Figure 3. Since refractive index is a function of critical angle, determination of the critical angle gives rise to determination of the refractive index of the sample, which is indicative of one or more sample properties, from which further qualitative and quantitative analyses about the quality of the sample may be made.

In accordance with fixed optic critical angle sensor 130, when light rays are directed to the sample at angles of incidence smaller than the critical angle, a portion of the light is refracted into the sample, resulting in an overall loss. At angles of incidence larger than the critical angle, total internal reflection occurs, and the full intensity of the light is reflected off the sample. The critical angle, and consequently the refractive index, may be then determined by measuring the intensities of the reflected light rays, and detecting a transition from a high intensity to a low intensity.

Fixed optic critical angle sensor 130 thus detects critical angle to find the sample's refractive index, as shown in Equation 1 below, where $n_2$ is the index of refraction of the medium of the sample, $n_1$ is the index of refraction of the medium of origin and $\theta_c$ is the critical angle.

$$n_2 = (n_1)(\sin \theta_c). \qquad \text{Eq. 1}$$

As shown in Figure 3, a light source 152 emits light rays, 154, 156, and 158 toward a mirrored surface 160. The light rays 154, 156 and 158 then travel in the direction of a sensing surface 162 which forms the interface between fixed optic critical angle sensor 130 and the sample. Thus, the sensing surface 162 is in direct contact with the sample.

The light rays 154, 156 and 158 strike sensing surface 162 at angles 164, 166 and 168, respectively. For angles of incidence smaller than the critical angle 166, a portion of the light is refracted into the sample, resulting in an overall loss. This is illustrated by refracted ray 170 which travels into the sample and reflected ray 172 which is reflected toward a photodetector 180.

At the critical angle 166, a light ray 174 reflects along sensing surface 162 at a 90° angle of refraction, minimizing the overall loss of light into the sample. Thus, the critical angle 166 can be measured as the angle measured between the incident light ray 176 and the normal to sensing surface 162. For angles of incidence larger than the critical angle 166, such as 168, the incident ray 178 is totally internally reflected, with no refracted component, and its full intensity is directed toward photodetector 180.

Light energy striking a pixel of photodetector 180 generates electron-hole pairs in the region under the pixel. The field generated by the bias on the pixel causes the electrons to collect in the element while the holes are swept into the substrate. Each sensing area in photodetector 180 thereby produces a signal on an output with a voltage that is proportional to the intensity of the light striking photodetector 180. This intensity and its corresponding voltage are at their maxima in the total internal reflection region.

It is desirable to have the light rays strike photodetector 180 at angles as close as possible to 90°. By shaping fixed optic critical angle sensor 130 such that light strikes the photodetector 180 at an angle close to 90°, photodetector 180 will have the maximum possible sensitivity. It should be understood, however, that many configurations of fixed optic critical angle sensor 130 may be employed consistent with the present invention.

As described, a range of angles of the reflected light rays are projected onto photodetector 180. The critical angle is marked by a transition from high to low. When fixed optic critical angle sensor 130 serves as quality control sensor 34 of Figure 1, the output, representing bit level data from photodetector 180, is transmitted to control system 14 for further qualitative and/or quantitative analysis.

Referring again to Figure 1, data elements pertaining to properties of the sample are transmitted from quality control sensor 34 to control system 14 via an interface 48. In accordance with the present invention, a variable control input 50 communicates with control system 14 via an electrical interface 52. Variable control input 50 thus may comprise a control knob to enable the end user to adjust the determined end point of quality. Variable control input 50 thus allows the end user to exercise discretion in determining what level of quality is deemed unacceptable. This determination may also be a consideration of such factors as the particular frying medium, as well as the food being fried.

When data transmitted via interface 48 to control system 14 is indicative of an unacceptably poor level of quality, control system 14 sends a signal via interface 54 to alarm 56, indicating that the cooking oil in vat 10 should be replaced. Alarm 56 may be an audio alarm, or a visual alarm, or a combination of both. In addition, control system 14 may open an electrically operated valve 58 via electrical interface 60 to allow the used cooking oil in vat 10 to flow through flow line 62 to a disposal unit 66. When a level sensor 68 indicates to control system 14 via electrical interface 70 that vat 10 is empty, control system 14 closes electrically operated valve 58. Control system 14 then opens electrically operated valve 72 via electrical interface 74 to allow fresh oil from oil supply 76 to proceed through flow line 78 into vat 10. When level sensor 68 communicates to control system 14 that vat 10 is full, control system 14 closes electrically operated valve 72.

Control system 14 also opens electrically operated valve 82 via electrical interface 84 to allow the sample to flow through flow line 86 into disposal unit 90. Control system 14 may also open electrically operated valve 92 via electrical interface 94 to allow one or more cleaning agents 95 to travel through flow line 96 to the sensing surface of quality control sensor 34.

In addition, data pertaining to the measurements of the sample's properties as well as the frequency and amounts of oil added to vat 10 may be transmitted via an interface 98 to a data storage unit 99. Control system 14 may also be communicably linked to a network to enable such data to be transmitted to a remote data storage unit. Recordation of such data at a remote location such as the restaurant's corporate headquarters, would enable automation of requests to ship additional units of oil, rather than storing large supplies of fresh oil locally, which is an inefficient use of space and capital.

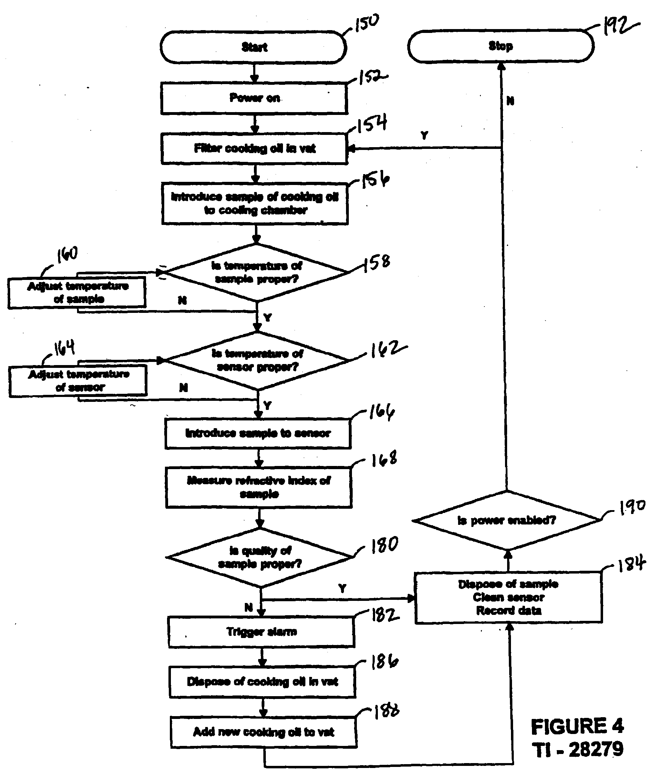

Reference is now made to Figure 4, in which a flow diagram of a method for sensing the quality of oil in accordance with the present invention is shown. Process flow begins in step 150, and the system is provided power in step 152. A vat of hot cooking oil may be optionally filtered to remove particulate matter in step 154. Utilizing appropriate valving, a sample of the hot cooking oil is introduced to a cooling chamber in step 156. The temperature of the sample is measured, and if the temperature exceeds a predetermined temperature at decision 158, the sample is allowed to cool to adjust the temperature in step 160, and its temperature is again monitored. When the temperature of the sample is approximately equivalent to a predetermined desired temperature, process flow proceeds to decision 162, where the temperature of a quality control sensor is monitored. If the temperature of the quality control sensor is below a certain predetermined temperature level, the temperature of the sensor is elevated in step 164 utilizing a heating coil, heat lamp or other heating device. When the temperature of the sensor is approximately equivalent to the temperature of the sample, the sample is introduced to a sensing surface of the quality control sensor in step 166.

Utilizing fixed optic surface plasmon resonance sensing or other fixed optic sensing techniques, the refractive index and other properties of the sample are measured in step 168. The measurements are compared to previously measured values in step 180. If the changes in the measurements indicate that the quality of the cooking oil has declined to an unacceptable level of quality pursuant to the user's preferences, an audio and/or visual alarm is triggered in step 182.

Optionally, the sub-par quality cooking oil in the vat may be disposed of in step 186, and fresh cooking oil may be added to the vat in step 188. The sample is disposed of in step 184, and cleaning agents are introduced to the cooling chamber and the sensor surface. Data pertaining to the refractive index measurements and the disposal or addition of cooking oil are recorded in memory, and may optionally be transmitted to a remote data storage system via a modem, infrared link or other means of communication. At decision 190, an inquiry is made as to whether power is still enabled. If power is off, process flow stops at 192. Otherwise, process flow continues, and the process repeats beginning at step 154.

While this invention has been described with reference to illustrative embodiments, this description is not intended to be construed in a limiting sense. Various modifications and combinations of the illustrative embodiments as well as other embodiments of the invention will be apparent to persons skilled in the art upon reference to the description.

**Claims**

1. A method of monitoring oil quality comprising:

   drawing a sample of oil from a container of oil;
   modifying the temperature of said sample of oil;
   passing said sample of oil onto a sensing surface of a fixed optic sensor; and
   optically sensing at least one property of said sample of oil.

2. The method as recited in Claim 1, wherein said step of passing said sample of oil onto a sensing surface of a fixed optic sensor comprises passing said sample of oil onto a sensing surface of a surface plasmon resonance sensor.

**3.** The method as recited in Claim 1, wherein said step of passing said sample of oil onto a sensing surface of a fixed optic sensor comprises passing said sample of oil onto a sensing surface of a refractometer.

**4.** The method as recited in Claim 1, wherein said step of sensing at least one property comprises sensing refractive index of said sample of oil.

**5.** The method as recited in any preceding claim further comprising:

the step of modifying the temperature of said fixed optic sensor.

**6.** The method as recited in Claim 5, wherein the step of modifying the temperature of said fixed optic sensor comprises heating said fixed optic sensor to substantially the temperature of the sample.

**7.** The method as recited in any preceding claim further comprising:

triggering an alarm based on said at least one property.

**8.** The method as recited in any preceding claim further comprising:

filtering said oil.

**9.** The method as recited in any preceding claim further comprising:

transmitting data related to said at least one property to a local data storage medium.

**10.** The method as recited in any preceding claim further comprising:

transmitting data related to said at least one property to a remote data storage medium.

**11.** The method as recited in any preceding claim further comprising:

cleaning said sensing surface of said fixed optic sensor.

**12.** The method as recited in any preceding claim further comprising:

mixing said sample with at least one reagent.

**13.** The method as recited in any preceding claim further comprising:

monitoring the level of said oil in said container.

**14.** The method as recited in any preceding claim further comprising:

automatically discarding said oil from said container based on said at least one property.

**15.** The method as recited in preceding claim further comprising:

automatically adding an amount of unused oil to said container of oil.

**16.** The method as recited in any preceding claim wherein said step of optically sensing at least one property of said oil with a fixed optic sensor comprises:

disposing said sensing surface at an angle to receive at least one light ray emitted from a light source; and
measuring intensity of a reflected component of said at least one light ray with a photodetector.

**17.** A system for monitoring oil quality, comprising:

a temperature control system arranged to be coupled to a container of oil;
a fixed optic sensor coupled to said temperature control system; and
a digital signal processing unit electrically connected to said fixed optic sensor.

**18.** The system as recited in Claim 17 further comprising:

a heating device thermally coupled to said fixed optic sensor.

**19.** The system as recited in Claim 17 or Claim 18 further comprising:

means for coupling a container of at least one reagent to said temperature control system.

**20.** The system as recited in any of Claim 17 to 19 further comprising:

means for coupling a container of at least one cleaning agent to said fixed optic sensor.

**21.** The system as recited in any of Claims 17 to 20 further comprising:

an alarm device electrically connected to said digital signal processing unit.

**22.** The system as recited in any of Claims 17 to 21 further comprising:

means for coupling a disposal unit to said fixed optic sensor.

23. The system as recited in any of Claims 17 to 22 further comprising:

means for coupling a disposal unit to said container of oil.

24. The system as recited in any of Claims 17 to 23 further comprising:

means for coupling a supply container of oil to said container of oil.

25. The system as recited in any of Claims 17 to 24 further comprising:

means for electrically coupling a variable control input to said digital signal processing unit.

26. The system as recited in any of Claims 17 to 25 further comprising:

means for coupling a local data storage medium electrically to said digital signal processing unit.

27. The system as recited in any of Claims 17 to 26 further comprising:

a remote data storage medium for receiving data elements transmitted from said digital signal processing unit via a network.

28. The system as recited in any of Claims 17 to 27 wherein said fixed optic sensor comprises:

a sensing surface disposed at an angle to receive at least one light ray emitted from a light source; and
a photodetector for measuring intensity of a reflected component of said at least one light ray.

29. A system for monitoring oil quality, comprising:

a temperature control system arranged to be coupled to a container of oil;
a fixed optic sensor coupled to said temperature control system;
a digital signal processing unit electrically connected to said fixed optic sensor;
a heating device thermally coupled to said fixed optic sensor;
means for coupling a container of at least one reagent to said temperature control system;
means for a container of at least one cleaning agent to said fixed optic sensor;
an alarm device electrically connected to said digital signal processing unit;
means for coupling a disposal unit to said fixed optic sensor;
means for coupling a disposal unit to said container of oil;
means for coupling a supply container of oil to said container of oil;
a variable control input electrically coupled to said digital signal processing unit;
a local data storage medium electrically coupled to said digital signal processing unit; and
means for transmitting data elements from said digital signal processing unit via a network to a remote data storage medium.

**FIGURE 1**
**TI - 28279**

**FIGURE 2**
**TI - 28279**

**FIGURE 3**
**TI - 28279**

9

FIGURE 4
TI - 28279

EP 1 004 872 A1

## EUROPEAN SEARCH REPORT

**European Patent Office**

**Application Number**

EP 99 20 3913

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 311 274 A (COLE JR CHARLES F) 10 May 1994 (1994-05-10) | 1,3-6,9, 12,13, 17-19, 25,26 | G01N21/55 G01N21/43 G01N33/03 |
| Y | | 2,7,8, 10,11, 14,16, 20-23, 27,28 | |
| A | * column 1, line 64 - column 2, line 8 * <br> * column 2, line 37 - line 43 * <br> * column 3, line 18 - line 20 * <br> * column 11, line 67 - column 12, line 58 * <br> * figures 4,5 * | 29 | |
| Y | US 5 835 645 A (JORGENSON RALPH C ET AL) 10 November 1998 (1998-11-10) <br> * column 1, line 16 - line 20 * <br> * column 7, line 62 - column 8, line 17 * | 2 | |
| Y | EP 0 635 714 A (NIPPON DENSO CO) 25 January 1995 (1995-01-25) <br><br> * column 3, line 40 - line 58 * <br> * column 10, line 26 - line 41 * <br> * column 11, line 5 - line 25 * <br> * column 19, line 18 - line 25 * <br> * column 19, line 51 - column 20, line 3 * <br> * figure 14 * | 7,8,11, 16,20, 21,28 | **TECHNICAL FIELDS SEARCHED (Int.Cl.7)** <br><br> G01N |
| | -/-- | | |

The present search report has been drawn up for all claims

| Place of search | Date of completion of the search | Examiner |
|---|---|---|
| BERLIN | 22 February 2000 | Navas Montero, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## European Patent Office

## EUROPEAN SEARCH REPORT

Application Number

EP 99 20 3913

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | EP 0 675 359 A (ON SITE ANALYSIS INC) 4 October 1995 (1995-10-04) * page 2, line 22 - line 25 * * page 2, line 53 - page 3, line 1 * * page 3, line 50 - page 4, line 18 * * page 4, line 36 - line 41 * * page 4, line 46 - line 48 * * figure 2 * | 10,14, 22,23,27 | |
| A | US 5 818 731 A (PAUL SATHEESH ET AL) 6 October 1998 (1998-10-06) * column 1, line 6 - line 10 * * column 3, line 28 - line 47 * * column 11, line 1 - line 13 * * column 15, line 50 - line 59 * * column 16, line 6 - line 13 * | 13,14 | |
| | | | TECHNICAL FIELDS SEARCHED (Int.Cl.7) |

The present search report has been drawn up for all claims

| Place of search | Date of completion of the search | Examiner |
|---|---|---|
| BERLIN | 22 February 2000 | Navas Montero, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 99 20 3913

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2000

| Patent document cited in search report | | Publication date | Patent family member(s) | | Publication date |
|---|---|---|---|---|---|
| US 5311274 | A | 10-05-1994 | NONE | | |
| US 5835645 | A | 10-11-1998 | US | 5647030 A | 08-07-1997 |
| | | | US | 5359681 A | 25-10-1997 |
| | | | AT | 160871 T | 15-12-1997 |
| | | | CA | 2153389 A | 21-07-1994 |
| | | | DE | 69407161 D | 15-01-1998 |
| | | | DE | 69407161 T | 26-03-1998 |
| | | | EP | 0678194 A | 25-10-1995 |
| | | | ES | 2114175 T | 16-05-1998 |
| | | | JP | 8505475 T | 11-06-1996 |
| | | | WO | 9416312 A | 21-07-1994 |
| EP 0635714 | A | 25-01-1995 | JP | 7063035 A | 07-03-1995 |
| | | | JP | 7072072 A | 17-03-1995 |
| | | | DE | 69416048 D | 04-03-1999 |
| | | | DE | 69416048 T | 01-07-1995 |
| | | | US | 5548393 A | 20-08-1996 |
| EP 0675359 | A | 04-10-1995 | US | 5537336 A | 16-07-1996 |
| | | | CA | 2145621 A | 01-10-1995 |
| | | | US | 5517427 A | 14-05-1996 |
| US 5818731 | A | 06-10-1998 | NONE | | |

For more details about this annex : see Official Journal of the European Patent Office, No. 12/82